# EUROPÄISCHE PATENTANMELDUNG

(11) **EP 3 127 999 A1**
(43) Veröffentlichungstag der Anmeldung: **08.02.2017**
(21) Anmeldenummer: 15179479.9
(22) Anmeldetag: 03.08.2015
(51) Int. Cl.: C12M 1/107, C12M 1/00, C12M 1/34

(54) **VERFAHREN ZUM BETREIBEN EINER ENERGIEERZEUGUNGSANLAGE MIT EINER BIOGASERZEUGUNGSEINHEIT UND EINEM BIOGASMOTOR**

(71) Anmelder: Innovative Biogas GmbH & Co. KG, 23909 Ratzeburg (DE)
(72) Erfinder: Liermann, Kai, 19294 Neu Kaliss (DE); Flaschka, Klaus, 23909 Ratzeburg (DE)
(74) Vertreter: Raffay & Fleck

(57) **Zusammenfassung**

Offenbart wird ein Verfahren zum Betreiben einer mit einer Biogaserzeugungseinheit und mit einem mit in der Biogaserzeugungseinheit gewonnenem Biogas betriebenen Biogasmotor gebildeten Energieerzeugungsanlage, wobei in einem Fermenter der Biogaserzeugungseinheit aus in dem Fermenter befindlichem Substrat Biogas gewonnen, dem Biogasmotor zugeführt und dort verbrannt wird zum Antreiben eines Generators zum Erzeugen von elektrischer Energie.

Mit der Erfindung wird eine Möglichkeit der Steuerung bzw. Regelung der Biogaserzeugung hinsichtlich der pro Zeiteinheit erzeugten Menge insbesondere für Kleinanlagen geschaffen, indem für die Steuerung der in der Biogaserzeugungseinrichtung erzeugten Biogasmenge in Abhängigkeit von einem zu erwartenden, durch die Energieerzeugungsanlage zu deckenden Energiebedarf in den Fermenter ein Steuergas so eingeleitet wird, dass es das darin enthaltene Substrat durchströmt, wobei als Steuergas ein die Fermentationsaktivitäten von in dem Fermenter vorhandenen Mikroorganismen hemmendes Gas verwendet wird, wenn ausgehend von einem momentanen Istzustand ein verringerter Energiebedarf zu erwarten ist, bei einem gegenüber dem momentanen Istzustand erhöht erwarteten Energiebedarf hingegen als Steuergas ein die Fermentationsaktivität der in dem Fermenter vorhandenen Mikroorganismen unterstützendes Gas verwendet wird.

## Beschreibung

Die vorliegende Erfindung betrifft ein Verfahren zum Betreiben einer mit einer Biogaserzeugungseinheit und mit einem mit in der Biogaserzeugungseinheit gewonnenem Biogas betriebenen Biogasmotor gebildeten Energieerzeugungsanlage, wobei in einem Fermenter der Biogaserzeugungseinheit aus in dem Fermenter befindlichem Substrat Biogas gewonnen, dem Biogasmotor zugeführt und dort verbrannt wird zum Antreiben eines Generator zum Erzeugen von elektrischer Energie.

Solche Verfahren sind aus dem Stand der Technik in vielfältiger Weise bekannt. Mit biogasbetriebene Energieerzeugungsanlagen, auch Biogasanlagen genannt, wird bereits heute, insbesondere im ländlichen Raum, ein beachtlicher Anteil an regenerativer Energie erzeugt. Die installierten Biogasanlagen sind dabei in der Regel in einer Größe dimensioniert, dass sie für die Energieversorgung mehrerer Haushalte, häufig ganzer Gemeinden, ausgelegt sind. Sie werden vielfach nach dem Prinzip der Kraft-Wärme-Kopplung betrieben, erzeugen also durch Verbrennen von Biogas neben elektrischem Strom zugleich auch nutzbare Wärme, die beispielsweise über Fernwärmeleitungen verteilt und den Verbrauchern zugeführt wird.

Die bestehenden vergleichsweise großen Anlagen sind mit entsprechend dimensionierter Anlagentechnik versehen, weisen voluminöse Fermenter auf und verfügen über entsprechend ausgelegte Biogasspeicher.

Derartige Großanlagen warten mit beachtlichen Leistungen auf. So lag im Jahr 2010 die durchschnittliche Leistung der installierten Biogasanlagen bei 380 kW. Besonders groß dimensionierte Anlagen können Leistungen ist deutlich in den Megawatt-Bereich hinein erzielen. Solche Anlagen sind als eigenständige Kleinkraftwerke zu betrachten und werden in Deutschland nach dem Erneuerbare-Energien-Gesetz (EEG) mit einer Abgabe für die Finanzierung der Energiewende, der EEG-Umlage, belegt. Diese Umlage wird in Deutschland für alle Elektrizitätsversorgungsunternehmen erhoben. Die Umlage wird auch für den Eigenverbrauch fällig, insoweit eine Leistungsgrenze der betriebenen Anlage 10 kW erreicht oder überschreitet.

Im Bereich der privaten Haushalte gibt es vermehrt Überlegungen, die Energieversorgung (Elektrizität, Heizung) autark zu gestalten und von den klassischen Energieträgern (Heizöl, Gas; kommerzielle Stromanbieter) abzukoppeln. Entsprechend haben viele Eigenheimbesitzer ihre Häuser mit Solaranlagen, Solarthermieanlagen, Pellet-Heizungen, Holzheizungen, Geothermie-Anlagen oder dergleichen ausgerüstet. Auf dem Gebiet der Versorgung einzelner Haushalte für den Eigenbedarf gibt es indes bisher noch keine tragende Umsetzung, die auf dem Konzept der Erzeugung und Nutzung von Biogas vor Ort in einem Kleinmaßstab basiert.

Hier setzen die Überlegungen der Erfinder an, die es sich zum Ziel gesetzt haben, auf der Erzeugung und Verwertung von Biogas basierende Energieerzeugungsanlagen im Kleinmaßstab zu entwickeln und für Privathaushalte oder kleinere und mittelständische Gewerbebetriebe zur Verfügung zu stellen. Im Hinblick der preislichen Attraktivität sollen nach den derzeitigen Rahmenbedingungen des EEG die Leistungen derartiger Anlagen unterhalb von 10 kW liegen, so dass keine EEG-Umlage auf die erzeugte Energie anfällt.

Die Entwicklung derartiger kleiner auf Biogas basierenden Energieerzeugungsanlagen bringt Anforderungen mit sich, die sich von den Anforderungen, die an Großanlagen gestellt sind, unterscheiden. Einerseits muss die gesamte Anlage kompakt gebaut sein, da sie selbstverständlich nicht den Flächenbedarf einer Großanlage aufweisen kann. Private Eigenheime haben ebenso wie kleinere und mittelständische Betriebe in der Regel nur begrenzte Grundflächen zur Verfügung, wobei diese auch nicht mit weitläufiger Anlagentechnik verbaut werden soll.

Darüber hinaus entfällt für eine entsprechende Energieerzeugungsanlage der bei Großanlagen wirksame, den Energiebedarf mittelnde Effekt eines Anschlusses einer Vielzahl von Haushalten. Die Anlage muss zuverlässig und anforderungskonform auf das spezifische Verbrauchsprofil des jeweils angeschlossenen Haushaltes oder Betriebes reagieren. Insbesondere kann, aufgrund des geringen Raumangebotes, in der Regel keine groß dimensionierte Speichereinheit für im Überschuss erzeugtes Biogas untergebracht werden. Entsprechend gilt es, den in der Biogaserzeugungseinheit der Energieerzeugungsanlage erzielten Biogasausstoß so zu beeinflussen und zu steuern, dass er dem durch das Verbrauchsprofil gegebenen Bedarf für die Verbrennung in dem Biogasmotor entsprechend geführt wird.

An dieser Stelle setzt die Erfindung an, die es sich zur Aufgabe gemacht hat, hier eine entsprechende Möglichkeit der Steuerung bzw. Regelung der Biogaserzeugung hinsichtlich der pro Zeiteinheit erzeugten Menge für solche Kleinanlagen zu schaffen.

Diese Aufgabe wird mit einem Verfahren gelöst, wie es in Anspruch 1 bestimmt ist. Vorteilhafte Weiterbildungen dieses Verfahrens sind in den Ansprüchen 2 bis 10 angegeben, wobei einzelne der dort genannten verfahrensmäßigen Maßnahmen auch losgelöst von einem in Anspruch 1 näher bezeichneten Verfahren durchgeführt werden können und insoweit eigenständige Lösungsaspekte darstellen können.

Erfindungsgemäß wird bei einem Verfahren nach der eingangs genannten Art für die Steuerung der in der Biogaserzeugungseinrichtung pro Zeiteinheit erzeugten Biogasmenge in Abhängigkeit von einem zu erwartenden, durch die Energieerzeugungsanlage zu deckenden Energiebedarf in den Fermenter ein Steuergas derart eingeleitet, dass es das darin enthaltene Substrat durchströmt. Dabei wird als Steuergas ein die Fermentationsaktivitäten von in dem Fermenter vorhandenen Mikroorganismen hemmendes Gas verwendet, wenn ausgehend von einem momentanen Ist-Zustand ein verringerter Energiebedarf zu erwarten ist. Wird hingegen gegenüber dem momentanen Ist-Zustand eine erhöhte Energiebedarf erwartet, wird als Steuergas ein die Fermentationsaktivität der in dem Fermenter vorhandenen Mikroorganismen unterstützendes Gas verwendet.

Im Rahmen der Erfindung wird also mit anderen Worten vorgeschlagen, die Aktivität der Mikroorganismen in dem Fermenter durch Zufuhr entsprechenden Steuergases zu beeinflussen, so dass die Mikroorganismen entsprechend in ihrer Fermentationsaktivität befördert oder gehemmt werden. Bei der Steuerung handelt es sich selbstverständlich nicht um eine instantan wirksame Steuerung. Allerdings haben die Erfinder herausgefunden, dass hier über eine Steuerzeitspanne von ca. 1 bis 4 Stunden eine sehr gute Führung des Integrationsprozesses erreicht werden kann. Diese Art der Steuerung eignet sich also sehr gut für die Anpassung der je Zeiteinheit erzeugten Menge an Biogas an typische, während des Tagesverlaufes wiederkehrend sich verändernde Energiebedarfsmuster. Derartige Muster sind beispielsweise typisch im Verlauf zwischen Tag und Nacht zu erkennen, wobei klassischerweise an Werktagen der Energiebedarf des morgens höher liegt als beispielsweise während der typischen Arbeitszeiten, am Abend noch einmal steigt, während er des nachts auf einem niedrigen Wert liegt. Allerdings sind hier die Bedarfskurven der unterschiedlichen Haushalte individuell verschieden, beispielsweise haben Haushalte, in denen mittags eine warme Mahlzeit zubereitet wird, in der Mittagsphase einen erhöhten Energiebedarf; solche Haushalte, bei denen die Bewohner tagsüber nicht zurückkehren, ihre Mittagsmahlzeit auswärts zu sich nehmen, haben zu dieser Zeit hingegen einen geringen Energiebedarf. Ein entsprechendes typischerweise wiederkehrendes Bedarfsprofil (sich unterscheidend zwischen Werktagen und freien Tagen, wie Sonn- und Feiertagen) kann für den mit einer entsprechenden Anlage versorgten Haushalt ermittelt und dann im Rahmen einer entsprechenden Programmierung der Anlage für die Umsetzung des Verfahrens berücksichtigt werden.

Der erfindungsgemäße Gedankenansatz, das gezielte Eindringen eines Steuergases in den Fermenter einer Biogaserzeugungseinheit zur Regulierung des Biogasausstoßes einzusetzen, ist bis dahin unbekannt. Zwar ist es durchaus bekannt, in Fermenter von Biogasanlagen Gas einzubringen, jedoch geschieht dies nicht zu Zwecken der Steuerung oder Beeinflussung des Biogasausstoßes im Sinne einer bedarfsangepassten Erhöhung oder Verringerung, sondern zu anderen Zwecken. So beschreibt die EP 2251408 A1 zwar das Einbringen eines CO₂-haltigen Abgase aus einer Biogasaufbereitungseinrichtung in einen Fermenter, allerdings geschieht dies zum Zwecke des Abfahrens des in dem Fermenter entstandenen Biogases, das mit dem eingespeisten CO₂ aus dem Fermenter gelöst und herausgespült wird. Der Gedanke einer Beeinflussung der Fermentationsaktivitäten der Mikroorganismen zum Zwecke einer Steuerung des pro Zeiteinheit erzeugten Biogasvolumens ist dort nicht beschrieben und für den Fachmann auch nicht nahegelegt.

Der Fermenter kann im Rahmen der Erfindung auch als Reaktor bezeichnet werden. Insbesondere ist es möglich, den Fermenter als mit einer Metallwand oder eine anderen starren Wandung umgebenen Behälter auszubilden, also ohne eine flexible Membran für eine Volumenvergrößerung beim Auffangen von Biogas, wie sie sonst häufig verwendet wird. Diese Maßnahme, die auch losgelöst von den weiteren Verfahrensmerkmalen der Erfindung eigenständig durchgeführt und insoweit als eigenständige Erfindung erkannt werden kann, führt dann nämlich dazu, dass bei einer erhöhten Biogasproduktion, die mengenmäßig die abgenommene Menge übersteigt, Biogas in dem Reaktorbehälter in einem Volumen oberhalb des Niveaus des Substrates aufgefangen wird unter Aufbau eines Speicherdrucks. Diese Druckanstieg führt dann dazu, dass - auch bei ansonsten unveränderten Bedingungen - die Biogasproduktion abnimmt, da wegen des erhöhten Drucks, insbesondere auch des Partialdrucks an Biogas, kaum mehr Biogas aus dem Substrat entweichen kann. Dort liegt dann gelöstes Biogas in größerer Konzentration vor, was letztlich auch die Mikroorganismen von der Produktion weiteren _Biogases abhält. Man erhält auf diese Weise eine Art rückgekoppelte Regulierung der pro Zeiteinheit erzeugten Menge an Biogas, die von der Abnahme, also dem Bedarf an Biogas abhängig ist.

Der Einfachheit halber wird nachstehend der Fermenter, der eben auch als Reaktor bezeichnet werden kann, weiterhin durchgehend Fermenter genannt, wobei stets die Ausbildung als Reaktor, insbesondere in einer Weise wie oben geschildert, mit als Gestaltungsmöglichkeit eingeschlossen sein soll.

Bei dem erfindungsgemäßen Verfahren kann mit Vorteil als die Fermentationsaktivitäten der in dem Fermenter vorhandenen Mikroorganismen hemmendes Steuergas ein CO₂-haltiges und/oder ein N₂-haltiges Gas oder ein solches Gasgemisch verwendet werden. Die in dem Fermenter ablaufenden Fermentationsprozesse werden durch das Einbringen derartigen Gases gehemmt und gebremst. Dadurch verringert sich der Ausstoß von Biogas, so dass über diese Maßnahme eine Anpassung an einen geringeren Energiebedarf erfolgen kann, indem entsprechend dem geringeren Verbrauch des Biogasmotors weniger Biogas zur Verfügung gestellt wird.

Als die Fermentationsaktivitäten der in dem Fermenter vorhandenen Mikroorganismen hemmendes Steuergas kann insbesondere Verbrennungsabgas des Biogasmotors verwendet werden. Dabei kann entsprechendes Abgas der Abgasleitung des Biogasmotors insbesondere in Strömungsrichtung gesehen vor einem Abgasreiniger und/oder Katalysator entnommen und in den Fermenter eingeführt werden. Diese Vorgehensweise hat den Vorteil, dass einerseits das Steuergas nicht gesondert beschafft werden muss, sondern das in dem Prozess ohnehin anfallende Abgas Verwendung findet. Darüber hinaus sind die Abgase heiß, so dass das Einbringen dieser Gase in den Fermenter nicht zu einer Abkühlung des für die Fermentation erwärmten, in dem Fermenter enthaltenen Substrates führt.

Als ein die Fermentationsaktivitäten der in dem Fermenter vorhandenen Mikroorganismen unterstützendes Steuergas kann ein O₂-haltiges Gas oder Gasgemisch, kann insbesondere Luft, verwendet werden. Hierbei wird mit Vorteil Luft aus der Umgebung des Fermenters genutzt, die durch die Abwärme des Fermenters und/oder die Abwärme des ebenfalls in diesem Bereich angeordneten Biogasmotors erwärmt ist, so dass auch die in den Fermenter eingespeiste Luft nicht zu einer Abkühlung des dort enthaltenen Substrats führt.

Mit Vorteil wird das Steuergas bodennah in den Ferment eingebracht. Auf diese Weise durchströmt das Gas den Fermenter in vertikaler Richtung und kommt so intensiv mit den in dem Fermenter enthaltenen Mikroorganismen in Kontakt, kann so deren Stoffwechselaktivitäten (Fermentationsaktivitäten) besonders gut und intensiv beeinflussen.

Für eine feine Verteilung des Steuergases wird dieses mit besonderem Vorteil in den Fermenter feinblasig eingeperlt. Hierzu können beispielsweise entsprechende Gasduschen, insbesondere im Bereich des Fermenterbodens, angeordnet und verwendet werden.

Um für die mit Energie zu versorgende Einheit, zum Beispiel eine privat genutzte Wohnimmobilie, oder auch eine Gewerbeimmobilie, sowohl elektrische Energie als auch Nutzwärme zur Verfügung zu stellen, wird die Energieerzeugungsanlage mit Vorteil nach dem Prinzip der Kraft-Wärme-Kopplung verwendet.

Die erfindungsgemäße Energieerzeugungsanlage ist insbesondere kompakt gebildet, wobei der Fermenter und der Biogasmotor in unmittelbarer räumlicher Nähe zueinander aufgebaut sind. Dies kann insbesondere in einer gemeinsamen gehäuseartigen Umhüllung erfolgen, zum Beispiel in einem Anlagencontainer. In einer solchen Anordnung kann zum Temperieren des Fermenters bzw. des darin enthaltenen Substrates insbesondere Abwärme des Biogasmotors verwendet werden, indem durch den Biogasmotor erwärmte Luft mit einem Gebläse gegen den Fermenter getrieben wird. Diese Art der Verwendung von ansonsten nicht mehr nutzbarer Abwärme führt zu einer weiteren Steigerung der Effizienz der Energieerzeugungsanlage. Während in den herkömmlichen Anlagenkonzepten häufig für das Temperieren der Fermenter die mit der Anlage gewonnene Nutzwärme (zum Beispiel die einem Wärmeträgermedium, wie insbesondere Wasser, bereits aufgeprägte Wärme) verwendet wird, wird hier die ansonsten nicht weiter verwendbare Prozessabwärme eingesetzt. Dabei kann die Anordnung des Fermenters und des Biogasmotors derart erfolgen, dass ein für die Kühlung des Biogasmotors ohnedies eingesetzter Lüfter so ausgerichtet wird, dass er die Abwärme des Biogasmotors, also die von dem Biogasmotor erwärmte Luft, in Richtung des Fermenters treibt. Diese Art der Erwärmung des Fermenters bzw. des in dem Fermenter enthaltenen Substrats ist insbesondere dann besonders effektiv, wenn der Fermenter an sich, bzw. eine Fermenterwand, gegen die die erwärmte Luft getrieben wird, aus einem Material mit guter Wärmeleitfähigkeit gebildet ist, insbesondere aus Metall. Für eine Regelung der Temperierung, die für das Einstellen und Aufrechterhalten einer vorgegebenen Fermentationstemperatur des Substrates erforderlich ist, kann bei dieser Lösung eine technisch einfache Realisierung gefunden werden, indem, wie dies eine vorteilhafte Weiterbildung der Erfindung vorsieht, mit einem quer zu dem Luftstrom der mit dem Gebläse gegen den Ferment der getriebenen, von dem Biogasmotor erwärmten Luft gerichteten, in seiner Stärke einstellbaren Querluftstrom gearbeitet wird. Dieser kann zum Beispiel mit einem weiteren Lüfter erzeugt werden, der mit einer Steuerung verbunden ist und von dieser gesteuert entsprechend angetrieben wird, um, wenn eine weitere Erwärmung des Fermenters nicht erforderlich ist, mit einem starken Querluftstrom die warme Luft, die über den Lüfter von dem Biogasmotor gegen den Fermenter getrieben wird, seitlich abzulenken. Soll der Fermenter hingegen weiter erwärmt werden, soll also dem Substrat Wärme zugeführt werden, so wird der Querluftstrom reduziert oder ganz abgeschaltet. Diese vorstehend beschriebene Lösung kann insbesondere auch unabhängig von der Verwendung eines Steuergases eingesetzt werden und stellt insoweit eine eigenständige Erfindung dar.

Die Verwendung eines Steuergases für eine Anpassung der Biogaserzeugung an einen Bedarfsverlauf ist, wie bereits vorstehend angeführt, keine solche zum Abfangen kurzfristiger Spitzen oder Dellen, sondern vielmehr eine Steuerung, die sich an einem mittelfristigen Verlauf orientiert und diesem folgend das Angebot an Biogas beeinflusst. Für die kurzfristige Leistungssteuerung und um insbesondere bei einem erhöhten plötzlich auftretenden Energiebedarf ohne Leistungseinbruch diesen decken zu können, wird mit der Erfindung eine weitere Maßnahme vorgeschlagen. Mit Vorteil kann hierbei der Biogasmotor über eine Biogasleitung und eine Luftzufuhrleitung mit den für die Verbrennung erforderlichen Medien Biogas und Luft versorgt werden, wobei zur Steuerung der Leistung des Biogasmotors bei einem erkannten erhöhten, von der Energieerzeugungsanlage kurzfristig zu deckenden Energiebedarf über eine zusätzliche, in der Luftzufuhrleitung mündende Speiseleitung der zugeführten Luft Biogas beigemengt werden kann. Hier wird also dem Biogasmotor in Reaktion auf durch entsprechende Sensorik in der Anlage festgestellte Verbrauchsspitzen eine erhöhte Menge an "Kraftstoff", also Biogas, zugeführt, indem nicht nur über die Biogasleitung als solche, sondern auch über die Luftzuführleitung dieser Treibstoff beigegeben wird. Der Biogasmotor fährt insoweit mit einem deutlich fetteren Gemisch, kann darüber deutlich mehr Leistung erbringen. Diese Art der Leistungssteigerung ist für Biogasmotoren, die einen Generator antreiben, eine gute und praktikable Wahl, da hierdurch die Drehzahl des Motors, die gleich gehalten werden muss, um über den Generator elektrische Energie mit einheitlicher Frequenz bereitzustellen, nicht beeinflusst wird. Auch diese Maßnahme ist wiederum eine solche, die nicht zwingend nur im Rahmen solcher Verfahren durchgeführt werden kann, bei denen für die Beeinflussung der Menge des erzeugten Biogases ein Steuergas in den Fermenter eingebracht wird. Sie stellt insoweit ebenfalls eine mögliche eigenständige Entwicklung und Erfindung dar.

Grundsätzlich ist es auch möglich, die Ausbeute an Biogas über eine Veränderung bzw. Anpassung der Temperatur des in dem Fermenter enthaltenen Substrates zu beeinflussen. Senkt man die Temperatur unterhalb eine optimale Fermentationstemperatur, bei der die Mikroorganismen die Biomasse mit der höchsten Intensität und Durchsatzrate verstoffwechseln, ab, so verringert sich die Ausbeute an Biogas. Hebt man die Temperatur wieder an, so steigt die Ausbeute dann erneut. Auch diese Effekt kann, insbesondere zusätzlich zu dem oben beschriebenen Eingriff über das Einbringen eines Steuergases in den Fermenter, genutzt werden, um Einfluss auf die Biogasausbeute zu nehmen. Allerdings ist auch eine solche Manipulation des Verfahrens träge, da das in der Regel große Volumen des Substrats für signifikante Temperaturänderungen deutlich Zeit benötigt.

Weitere Vorteile und Merkmale der Erfindung ergeben sich aus der nachfolgenden Beschreibung eines Ausführungsbeispiels anhand der beigefügten Figur. Es zeigt:

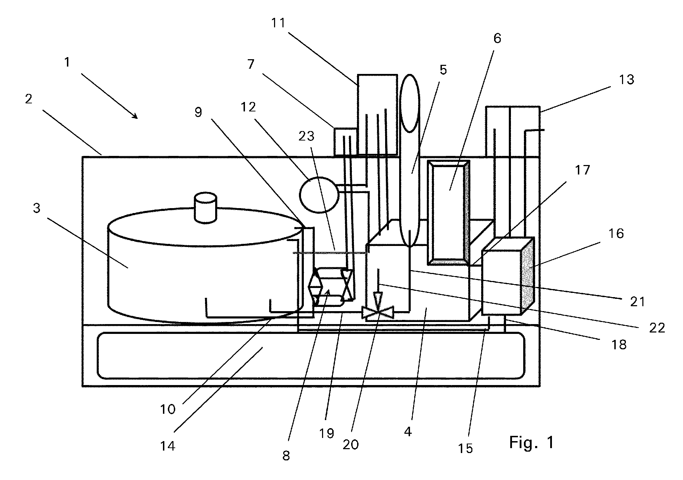

- Fig. 1: eine schematische Prinzipdarstellung einer nach dem erfindungsgemäßen Verfahren arbeitenden Energieerzeugungsanlage.

Bei der einzigen beigefügten Figur 1 handelt es sich um eine Prinzipdarstellung einer als Kompaktanlage gestalteten Energieerzeugungsanlage 1, die das erfindungsgemäße Verfahren umsetzt und nutzt.

Die Figur zeigt schematisch eine Energieerzeugungsanlage 1, deren wesentliche Komponenten in einem gemeinsamen Gehäuse in Form eines Containers 2 angeordnet sind. Wesentliche Komponenten der Energieerzeugungsanlage 1 sind dabei ein Fermenter 3 zur Erzeugung von Biogas sowie ein Biogasmotor 4, der über eine Verbrennung des in dem Fermenter erzeugten Biogases einen Generator zur Stromerzeugung antreibt und dessen Abwärme als Nutzwärme ausgenutzt wird. Die Abgase des Biogasmotors 4 werden über einen Schornstein 5 abgeführt und aus dem Container 2 abgeleitet, dann in die Umgebungsluft entlassen. In der Abgasführung ist, hier nicht näher dargestellt, eine Abgasreinigung, insbesondere ein Katalysator, angeordnet.

Die verschiedenen Komponenten der Energieerzeugungsanlage 1 werden über eine Steuerung 6 zum Betrieb der Energieerzeugungsanlage 1 gesteuert.

Verschiedene Anschlüsse sind zu erkennen, über die unterschiedliche Medien in die Energieerzeugungsanlage 1 eingespeist bzw. aus dieser abgeführt werden können. So ist ein mit zwei Leitungen versehener Substratanschluss 7 zu erkennen, über den frisches Substrat zugeführt und über eine Pumpeneinrichtung 8 in das Innere des Fermenters 3 befördert werden kann. Dies geschieht über eine von oben in den Fermenter 3 geführte, oberhalb des Niveaus des in diesem eingefüllten Substrats mündende Speiseleitung 9. Über eine zweite in dem Substratanschluss 7 vorgesehene Leitung kann verbrauchtes Substrat mit Gärrückständen abgepumpt werden. Dies geschieht über eine Abzugleitung 10 im Bodenbereich des Fermenters 3.

In einem Energieanschluss 11 sind eine elektrische Ausgangsleitung zum Abführen der mittels des hier nicht näher dargestellten, an den Biogasmotor 4 angeschlossenen Generators erzeugten elektrischen Stroms, sowie eine Vorlauf- und eine Nachlaufleitung eines Wärmeträgermediums zur Abfuhr der Nutzwärme zusammengefasst. In der Vorlaufleitung des Wärmeträgermediums ist ein Ausgleichsbehälter 12 angeordnet.

Schließlich ist ein Gasanschluss 13 vorgesehen, in dem eine Anschlussleitung zur externen Zuführung von (Bio-)Gas, ein Anschluss zum Abführen von Biogas sowie eine Überdruckabblasleitung (für das Abblasen von Biogas in einem Notfall) vorgesehen sind. Über die Anschlussleitung zum Zuführen von (Bio-)Gas kann die Energieerzeugungsanlage 1 mit (Bio-)Gas betankt werden, z.B. für ein Anfahren oder auch, wenn in dem Fermenter 3 nicht ausreichend Biogas für den Betrieb der Anlage erzeugt werden kann. Der Anschluss zum Abführen von Biogas erlaubt eine Entnahme von überschüssig erzeugtem Biogas mit z.B. einem Tankwagen.

In einem separaten Bauraum durch eine Bodenwand vor dem restlichen Bauraum abgetrennt, ist ein interner Gasspeicher 14 angeordnet, in welchem erzeugtes Biogas zwischengespeichert werden kann.

In dem Fermenter 3 entstehendes Biogas wird über eine Gasabzugleitung 15 an einen Gasverteiler 16 gegeben, der über eine Gaszufuhrleitung 17 den Biogasmotor 4 mit Biogas als Brennstoff versorgt, über den aber auch der Gasanschluss 13 angeschlossen ist und der mit einer Überführungsleitung 18 mit dem Gasspeicher 14 verbunden ist. Vor einer Überführung in den Gasspeicher 14 kann das Biogas mit einer hier nicht näher dargestellten Biogasverflüssigkungseinrichtung verflüssigt werden.

Für die erfindungsgemäße Führung des Verfahrens ist eine im Bodenbereich des Fermenters 3 über Gaseinperleinrichtungen mündende Steuergasspeiseleitung 19 vorgesehen, über die für eine Beeinflussung der Fermentationsaktivität der Mikroorganismen Steuergas in den Fermenter 3 eingeperlt werden kann. Über ein Umschaltventil 20 kann in die Steuergasspeiseleitung 19 wahlweise unterschiedliches Steuergas eingespeist werden. Es können für ein Hemmen der Fermentationsaktivitäten der in dem Fermenter 3 in dem Substrat enthaltenen Mikroorganismen über eine an die in den Schornstein 5 mündende Abgasleitung des Biogasmotors 4 angeschlossene Abzweigleitung 21 Verbrennungsabgase des Biogasmotors 4 in die Steuergasspeiseleitung 19 gegeben werden. Alternativ kann über eine Ansaugleitung 22 Luft in die Steuergasspeiseleitung gegeben werden, um die Fermentationsaktivität der Mikroorganismen zu befördern, die Biogasausbeute zu erhöhen. Für die entsprechende Einspeisung des jeweiligen Steuergases ist, hier nicht dargestellt, in der Steuergasspeiseleitung 19 eine entsprechende Gaspumpe, bzw. ein Kompressor angeordnet.

Das Umschaltventil 20 ist dabei mit der Steuerung 6 verbunden, wobei die Steuerung 6 abhängig vom einer zuvor festgestellten und festgelegten Bedarfskurve der von der Energieerzeugungsanlage 1 bereitzustellenden Energie das Umschaltventil 20 derart steuert, dass, wenn ein geringerer Energiebedarf bevorsteht, über die Abzweigleitung 21 Verbrennungsabgase des Biogasmotors 4 in den Fermenter eingespeist werden, dass, wenn eine Erhöhung des Energiebedarfs ansteht, über die Ansaugleitung 22 Luft angesagt und in den Fermenter 3 eingeperlt wird. Auf diese Weise wird eine Regulierung der erzeugten Biogasmenge pro Zeiteinheit in Anpassung an den Bedarf erzielt.

Um kurzfristige Bedarfsspitzen ohne ein Zusammenbrechen der Ausgangsleistung zu bedienen, ist eine Einspritzleitung 23 vorgesehen, über die unmittelbar aus dem Volumen des Fermenters 3 Biogas in eine Luftzufuhrleitung des Biogasmotors 4 gegeben und so für eine Leistungssteigerung zusätzlich der Verbrennung zugeführt werden kann. Eine solche Einspritzleitung 23 kann selbstverständlich auch ausgehend vom dem Gasverteiler 16 zu der Luftzufuhrleitung geführt und kann über diesen Weg dem Biogasmotor 4 zusätzlich Biogas zur Verfügung gestellt werden. In der Einspritzleitung 23 ist ein - hier nicht gezeigtes - Ventil enthalten, welches von der Steuerung 6 bei Bedarf geöffnet wird, um so die zusätzliche Leistungssteigerung des Biogasmotors 4 bewirken zu können.

Nicht näher dargestellt ist hier ein weiteres Element: Über einen Lüfter wird von dem Biogasmotor 4 erzeugte Warmluft direkt auf den Fermenter 3 geblasen. Der Fermenter 3 ist mit seiner Außenwand aus einem Metall, insbesondere aus Edelstahl, gebildet, so dass gegen diese geleitete Warmluft die Fermenter Wand und damit auch das in dem Fermenter enthaltene Substrat gut erwärmen kann. Ein Querlüfter erzeugt einen Querluftstrom, um über die Stärke desselben eine variable Ablenkung des Warmluftstromes vornehmen und darüber die Erwärmung des Fermenters steuern zu können. Dieser Querlüfter ist von der Steuerung 6 angesteuert und wird entsprechend in seiner Drehzahl verändert und angepasst zum Aufrechterhalten der für eine optimale Fermentation erforderlichen Substrattemperatur.

### Bezugszeichenliste

- 1: Energieerzeugungsanlage
- 2: Container
- 3: Fermenter
- 4: Biogasmotor
- 5: Schornstein
- 6: Steuerung
- 7: Substratanschluss
- 8: Pumpeneinrichtung
- 9: Speiseleitung
- 10: Abzugleitung
- 11: Energieanschluss
- 12: Ausgleichsbehälter
- 13: Gasanschluss
- 14: Gasspeicher
- 15: Gasabzugleitung
- 16: Gasverteiler
- 17: Gaszufuhrleitung
- 18: Überführungsleitung
- 19: Steuergasspeiseleitung
- 20: Umschaltventil
- 21: Abzweigleitung
- 22: Ansaugleitung
- 23: Einspritzleitung

## Patentansprüche

1. Verfahren zum Betreiben einer mit einer Biogaserzeugungseinheit und mit einem mit in der Biogaserzeugungseinheit gewonnenem Biogas betriebenen Biogasmotor gebildeten Energieerzeugungsanlage, wobei in einem Fermenter der Biogaserzeugungseinheit aus in dem Fermenter befindlichem Substrat Biogas gewonnen, dem Biogasmotor zugeführt und dort verbrannt wird zum Antreiben eines Generators zum Erzeugen von elektrischer Energie, **dadurch gekennzeichnet, dass** für die Steuerung der in der Biogaserzeugungseinrichtung erzeugten Biogasmenge in Abhängigkeit von einem zu erwartenden, durch die Energieerzeugungsanlage zu deckenden Energiebedarf in den Fermenter ein Steuergas so eingeleitet wird, dass es das darin enthaltene Substrat durchströmt, wobei als Steuergas ein die Fermentationsaktivitäten von in dem Fermenter vorhandenen Mikroorganismen hemmendes Gas verwendet wird, wenn ausgehend von einem momentanen Istzustand ein verringerter Energiebedarf zu erwarten ist, bei einem gegenüber dem momentanen Istzustand erhöht erwarteten Energiebedarf hingegen als Steuergas ein die Fermentationsaktivität der in dem Fermenter vorhandenen Mikroorganismen unterstützendes Gas verwendet wird.

2. Verfahren nach Anspruch 1, **dadurch gekennzeichnet, dass** als die Fermentationsaktivitäten der in dem Fermenter vorhandenen Mikroorganismen hemmendes Steuergas ein CO₂- und/oder N₂-haltiges Gas oder Gasgemisch verwendet wird.

3. Verfahren Anspruch 2, **dadurch gekennzeichnet, dass** als die Fermentationsaktivitäten der in dem Fermenter vorhandenen Mikroorganismen hemmendes Steuergas Verbrennungsabgas des Biogasmotors verwendet wird.

4. Verfahren nach einem der vorhergehenden Ansprüche, **dadurch gekennzeichnet, dass** als die Fermentationsaktivitäten der in dem Fermenter vorhandenen Mikroorganismen unterstützendes Steuergas ein O₂-haltiges Gas oder Gasgemisch verwendet wird.

5. Verfahren nach einem der vorhergehenden Ansprüche, **dadurch gekennzeichnet, dass** das Steuergas bodennah in den Fermenter eingebracht wird.

6. Verfahren nach einem der vorhergehenden Ansprüche, **dadurch gekennzeichnet, dass** das Steuergas in den Fermenter feinblasig eingeperlt wird.

7. Verfahren nach einem der vorhergehenden Ansprüche, **dadurch gekennzeichnet, dass** die Energieerzeugungsanlage nach dem Prinzip der Kraft-Wärme-Kopplung zur gleichzeitigen Gewinnung von elektrischer Energie und Nutzwärme betrieben wird.

8. Verfahren nach einem der vorhergehenden Ansprüche, **dadurch gekennzeichnet, dass** zum Temperieren des Fermenters Abwärme des Biogasmotors verwendet wird, indem durch den Biogasmotor erwärmte Luft mit einem Gebläse gegen den Fermenter getrieben wird.

9. Verfahren nach Anspruch 8, **dadurch gekennzeichnet, dass** die Temperierung des Fermenters über einen quer zu dem Luftstrom der mit dem Gebläse gegen den Fermenter getriebenen, von dem Biogasmotor erwärmten Luft gerichteten, in seiner Stärke einstellbaren Querluftstrom gesteuert wird.

10. Verfahren nach einem der vorhergehenden Ansprüche, **dadurch gekennzeichnet, dass** der Biogasmotor über eine Biogasleitung und eine Luftzufuhrleitung mit den für die Verbrennung erforderlichen Medien Biogas und Luft versorgt wird, wobei zur Steuerung der Leistung des Biogasmotors bei einem erkannten erhöhten, vom der Energieerzeugungsanlage kurzfristig zu deckenden Energiebedarf über eine zusätzliche, in der Luftzufuhrleitung mündende Speiseleitung der zugeführten Luft Biogas beigemengt wird.
